# FASCICULE DE BREVET EUROPEEN

(11) **EP 1 224 196 B1**
(45) Date de publication et mention de la délivrance du brevet: **14.11.2007**
(21) Numéro de dépôt: 00971502.0
(22) Date de dépôt: 25.10.2000
(51) Int. Cl.: C07H 15/24, C07H 15/26, C07H 15/203, C12Q 1/25

(54) **SUBSTRAT ENZYMATIQUE, PROCEDE DE SYNTHESE ET UTILISATIONS**
ENZYMATISCHES SUBSTRAT, VERFAHREN ZUR HERSTELLUNG UND VERWENDUNG DESSELBEN
ENZYMATIC SUBSTRATE, SYNTHESIS METHOD AND USES

(30) Priorité: 28.10.1999 FR 9913756
(43) Date de publication de la demande: 24.07.2002
(73) Titulaire: BIO MERIEUX, 69280 Marcy l'Etoile (FR)
(72) Inventeur: ARMSTRONG, Lyle, Ashington, Nottinghamshire NE63 8AE (GB); JAMES, Arthur, Newcastle Upon Tyne and Wear NE2 1HR (GB)
(74) Mandataire: Guerre, Dominique
(86) Numéro de dépôt international: PCT/FR2000/002971
(87) Numéro de publication internationale: WO 2001/030794

(56) Documents cités:
- US-A- 5 358 854
- US-A- 5 364 767
- TOKUTAKE S ET AL: "Glycosides having chromophores as substrates for sensitive enzyme analysis. I. Synthesis of phenolindophenyl-.beta.-D-glucopyranosides as substrates for.beta.-glucosidase" CHEM. PHARM. BULL. (CPBTAL,00092363);1990; VOL.38 (1); PP.13-18, XP002137365 Kikkoman Corp.;Res. Dev. Div.; Chiba; 278; Japan (JP)
- DATABASE WPI Section Ch, Week 198912 Derwent Publications Ltd., London, GB; Class B03, AN 1989-090852 XP002137367 & JP 01 042497 A (KIKKOMAN CORP), 14 février 1989 (1989-02-14)
- DATABASE WPI Section Ch, Week 198909 Derwent Publications Ltd., London, GB; Class B03, AN 1989-066064 XP002137448 & JP 01 019088 A (KIKKOMAN CORP), 23 janvier 1989 (1989-01-23)

## Description

La présente invention concerne une nouvelle famille de substrats enzymatiques et leurs applications notamment pour la détection de micro-organismes.

La détection et l'identification de bactéries est très importante en médecine ou dans l'industrie alimentaire sachant que ces micro-organismes peuvent non seulement se révéler être des agents pathogènes, mais également mettre en évidence certains types de contaminations.

Les méthodes récentes développées dans ce but font appel à l'utilisation de composés colorés ou fluorescents et les applications de ces composés ont été décrites par exemple dans les revues de Manafi M et al., Microbiological Reviews, 55(3), p 335-348, 1991 ou plus récemment Manafi M., De Ware(n) Chemicus, 28, p 12-17, 1998.

De manière générale, quatre groupes de composés peuvent être distingués :
- les marqueurs fluorescents comme l'acridine orange qui produisent une augmentation de fluorescence quand le marqueur est adsorbé sur les acides nucléiques ou les protéines des cellules des micro-organismes;
- les indicateurs pH dépendants comme l'acridine ou la 7-hydroxycoumarine qui varient en intensité ou en spectre de couleur ou fluorescence en fonction du pH et donc de l'activité biochimique des micro-organismes;
- les composés sensibles à la nature oxydo-réductrice du milieu comme le bleu de méthylène et qui produisent une couleur sous l'effet d'un milieu réducteur ;
- les substrats d'enzymes colorés ou fluorescents qui produisent respectivement une couleur ou de la fluorescence sous l'effet d'une hydrolyse en présence d'une enzyme spécifique d'un micro-organisme ou d'un groupe de micro-organismes.

Parmi cette dernière catégorie, un certain nombre de substrats ont été décrits, qui permettent notamment la détection de la β-D-glucosidase ou β-D-galactosidase qui sont des marqueurs taxinomiques importants pour les micro-organismes et en particulier les *Enterobacteriaceae.* Parmi ces substrats, on peut citer les dérivés de l'ortho-nitrophényle qui produisent de l'o-nitrophénol jaune après hydrolyse ou des substrats fluorescents comme les dérivés de la fluorescéine ou de la 4-méthylumbelliferone qui produisent un marqueur fluorescent. Une limitation importante de ces substrats est le phénomène de diffusion dans le milieu de culture ce qui rend plus difficile la distinction entre la colonie et le bruit de fond.

Pour résoudre ce problème, d'autres substrats ont été employés qui donnent un produit insoluble après hydrolyse. Ces substrats restent localisés autour de la colonie bactérienne sans diffuser sur le support de culture ce qui facilite l'interprétation du test.

Parmi ces substrats, les dérivés de l'indoxyl (voir par exemple Kodaka et al., J. Clin Microbiol., 33, p 199-201, 1995 ou les brevets US 5 358 854 et US 5 364 767) ou de l'esculétine (voir par exemple WO 97/41138) ont été développés. Pour les premiers la difficulté de synthèse rend le coût très élevé limitant le développement d'applications industrielles à grande échelle et de plus, ces substrats sont sensibles aux conditions d'incubation du milieu. Pour les deuxièmes, l'obligation de rajouter dans le milieu un agent complexant à base de fer pour avoir une réaction colorée pose problème pour certains micro-organismes car il peut interférer avec leur métabolisme et notamment celui étudié.

La présente invention décrit une nouvelle famille de substrats enzymatiques qui ne présente pas les inconvénients précités puisque la synthèse de ces composés est simple à mettre en oeuvre, les substrats sont insolubles après hydrolyse, ne diffusent pas dans le milieu et ils ne nécessitent pas l'addition d'agent complexant comme le fer ou de conditions d'oxydo-réduction particulières.

Un objet de la présente invention est de décrire une famille de composés de formule générale (I) : 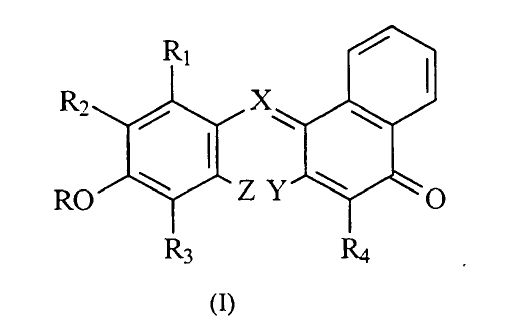 dans laquelle
X représente un atome d'azote ou un atome de carbone substitué par un groupement phényle, ledit groupement phényle étant éventuellement substitué en position méta ou para,
Y et Z représentent un atome d'hydrogène lorsque X représente un atome de carbone ou Y et Z représentent ensemble une liaison éther, thioéther ou amine éventuellement substituée par un groupement alkyle ou aryle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non,
R₃ et R₄ représentent chacun indépendamment l'un de l'autre H, Cl, F, I, Br et peuvent être identiques ou différents,
R représente un groupement tel que la liaison O-R est hydrolysable par une enzyme.

Les enzymes d'intérêt dans la présente invention sont les enzymes dont la présence donne une information permettant la détection et/ou l'identification et/ou la quantification d'un ou de plusieurs micro-organismes ou d'un analyte comme une protéine, un peptide, un acide nucléique. En particulier, l'enzyme est de la famille des osidases comme la β-glucuronidase, la β-galactosidase, la 6-phosphogalactohydrolase, l'α-galactosidase, l'α-amylase, l'α-glucosidase, la β-glucosidase, les hexosaminidases comme la N-acétyl-β-glucosaminidase ou la N-acétyl-β-galactosaminidase, de la famille des estérases, des lipases, des phosphatases, des sulfatases, des DNases, des peptidases et des protéases. Préférentiellement l'enzyme est de la famille des osidases comme la β-D-glucosidase, β-glucuronidase ou la β-D-galactosidase, de la famille des sulfatases, phosphatases, estérases.

Le groupement R est choisi en fonction de l'enzyme à détecter. R est, par exemple, un résidu de type sucre α- ou β- comme les dérivés du xylose, glucose, galactose, acide glucuronique, glucosamine ou un phosphate, un sulfate, un carboxylate (R₆COO-) dans lequel R₆ est un groupement alkyle ayant de un à 16 atomes de carbone, un nucléotide, un peptide. Préférentiellement le groupement R est le β-D-glucose ou le β-D-galactose, β-D-glucuronate, un phosphate, un sulfate, un acétate.

Quand R₁ et R₂ représentent ensemble un noyau benzénique fusionné, on entend la structure (Ia) suivante qui indique la formule développée pour R₁ et R₂ :

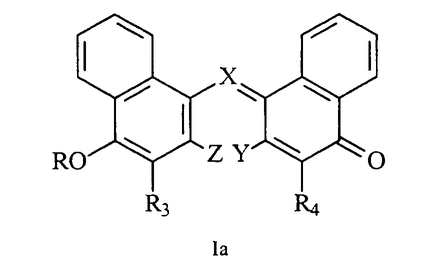

Quand Y et Z représentent ensemble une liaison éther, on entend une liaison telle que représentée dans le formule générale (Ib) :

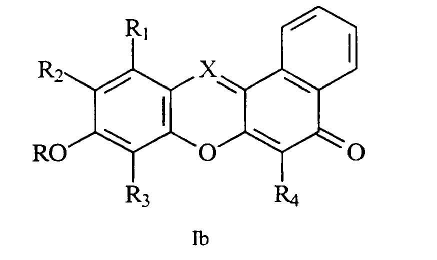

Quand Y et Z représentent ensemble une liaison thioéther, on entend une liaison telle que représentée dans le formule générale (Ic):

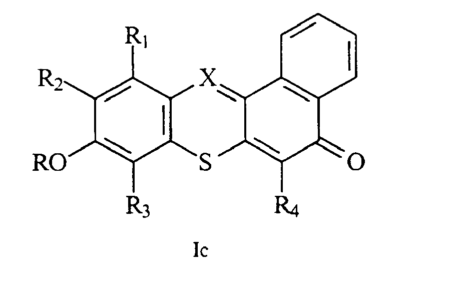

Quand Y et Z représentent ensemble une liaison amine éventuellement substituée par un groupement aryle ou alkyle, on entend une liaison telle que représentée dans le formule générale (Id) : 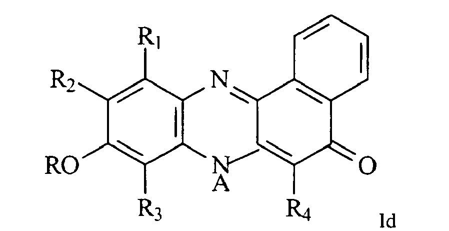 dans laquelle A représente un groupe aryle ou alkyle,

Des substrats particuliers de la formule générale (I) sont donnés dans les formules générales (II), (III), (IVa), (IVb) et (IVc) indiquées ci-dessous : 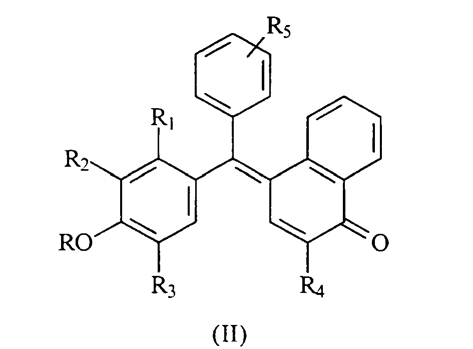 formule générale (II) dans laquelle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non. Préférentiellement R₁ et R₂ ensemble représentent un noyau benzénique fusionné non substitué.
R₃ et R₄ représentent chacun indépendamment l'un de l'autre H, Cl, F, I, Br et peuvent être identiques ou différents. Préférentiellement R₃ et R₄ représentent H,
R₅ représente H, NO₂, CF₃, CN, OCH₃, F, I, Cl, Br, SO₃H, CO₂H en position méta ou para. Préférentiellement R₅ représente H,
R représente un groupement tel que la liaison O-R est hydrolysable par une enzyme. En particulier R représente un résidu de type sucre α- ou β-, préférentiellement le β-D-glucose ou le β-D-galactose ou un acétate, un sulfate. 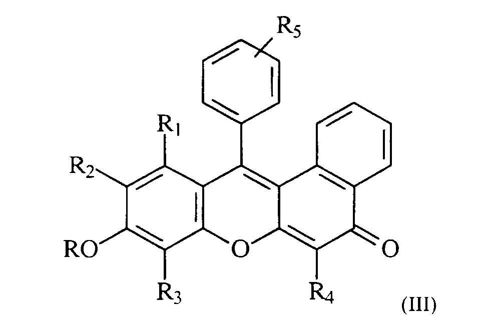 formule générale (III) dans laquelle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non. Préférentiellement R₁ et R₂ ensemble représentent un noyau benzénique fusionné non substitué.
R₃ et R₄ représentent chacun indépendamment l'un de l'autre H, Cl, F, I, Br et peuvent être identiques ou différents. Préférentiellement R₃ et R₄ représentent H.
R₅ représente H, NO₂, CF₃, CN, OCH₃, F, I, Cl, Br, CO,H, SO₃H en position méta ou para. Préférentiellement R₅ représente H.
R représente un groupement tel que la liaison O-R est hydrolysable par une enzyme. En particulier, R représente un résidu de type sucre α- ou β-, préférentiellement le β-D-glucose ou le β-D-galactose. 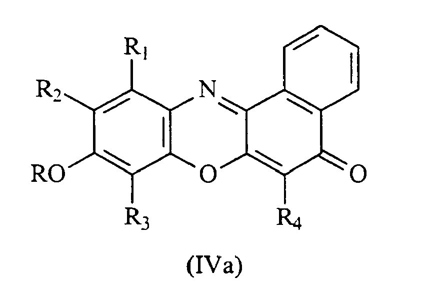 formule générale (IVa) dans laquelle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non. Préférentiellement R₁ représente H et R₂ représente Cl.
R₃ et R₄ représentent chacun indépendamment l'un de l'autre H, Cl, F, I, Br et peuvent être identiques ou différents. Préférentiellement R₃ et R₄ représentent H.
R représente un groupement tel que la liaison O-R est hydrolysable par une enzyme. En particulier, R représente un résidu de type sucre α- ou β-, préférentiellement le β-D-glucose ou le β-D-galactose ou un acétate ou un sulfate. 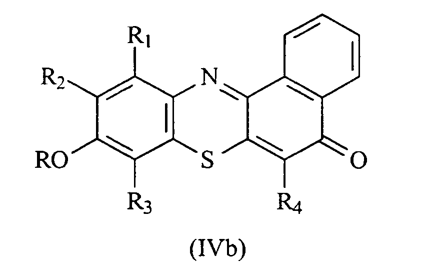 formule générale (IVb) dans laquelle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non. Préférentiellement R₁ représente H et R₂ représente Cl.
R₃ et R₄ représentent chacun indépendamment l'un de l'autre H, Cl, F, I, Br et peuvent être identiques ou différents. Préférentiellement R₃ et R₄ représentent H.
R représente un groupement tel que la liaison O-R est hydrolysable par une enzyme. En particulier, R représente un résidu de type sucre α- ou β-, préférentiellement le β-D-glucose ou le β-D-galactose ou un acétate ou un sulfate. 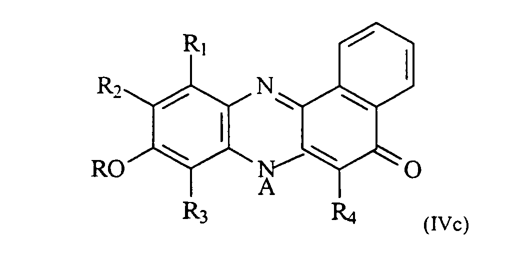 formule générale (IVc) dans laquelle,
A représente un groupe aryle ou alkyle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non. Préférentiellement R₁ représente H et R₂ représente Cl.
R₃ et R₄ représentent chacun indépendamment l'un de l'autre H, Cl, F, I, Br et peuvent être identiques ou différents. Préférentiellement R₃ et R₄ représentent H.
R représente un groupement tel que la liaison O-R est hydrolysable par une enzyme. En particulier, R représente un résidu de type sucre α- ou β-, préférentiellement le β-D-glucose ou le β-D-galactose ou un acétate ou un sulfate.

L'invention concerne également un procédé de synthèse du substrat enzymatique de formule générale (I) dans lequel on prépare un intermédiaire de formule générale (V) 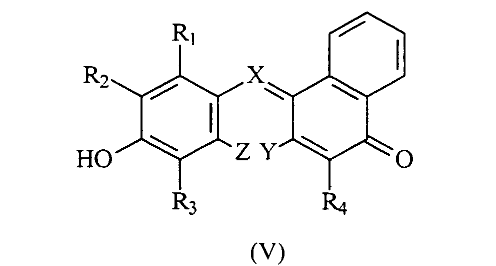 dans laquelle
X représente un atome d'azote ou un atome de carbone substitué par un groupement phényle, ledit groupement phényle étant éventuellement substitué en position méta ou para,
Y et Z représentent un atome d'hydrogène lorsque X représente un atome de carbone ou Y et Z représentent ensemble une liaison éther, thioéther ou amine éventuellement substituée par un groupement alkyle ou aryle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non,
R₃ et R₄ représentent chacun indépendamment l'un de l'autre H, Cl, F, I, Br et peuvent être identiques ou différents.
et on greffe sur l'hydroxyle un groupement R convenablement protégé.

En particulier le greffage s'effectue par une réaction de glycosylation, estérification, phosphorylation.

Dans le cas de la glycosylation, il est nécessaire de protéger les groupements hydroxyles du sucre comme par exemple avec un groupement acétyle. Les dérivés tétraacétylés du β-D-glucoside ou β-D-galactoside sont obtenus à partir du dérivé correspondant α-acétobromohexose.

La déprotection des groupements hydroxyles du sucre est réalisée après le greffage en présence d'un agent alcalin comme le méthoxyde de sodium dans le méthanol pour un dérivé acétylé. Les conditions de glycosylation sont choisies par l'homme du métier et en particulier par action de l'hydroxyde de potassium dans l'acétone.

La formation d'esters organiques comme l'acétate est réalisée en faisant réagir sur le dérivé de formule générale (V), l'anhydride acétique à froid dans la pyridine. La formation d'ester du type CH₃(CH₂)ₙ COOR est réalisée en faisant réagir un dérivé chlorure d'acide dans un mélange de solvant type pyridine / diméthylformamide, éventuellement en présence d'un catalyseur comme la 4-diméthylaminopyridine.

La formation de phosphate est réalisée en traitant le dérivé de formule générale (V) en présence de POCl₃ en présence de pyridine.

La formation de sulfate est réalisée en traitant le dérivé de formule générale (V) par action de l'acide chlorosulfonique à froid dans la pyridine. Les substrats de la présente invention dans le cas des phosphates et sulfates sont sous forme de sel comme un sel de potassium ou de sodium.
Avantageusement, on prépare un intermédiaire répondant à l'une quelconque des formules (Va), (Vb) et (Vc) suivantes :

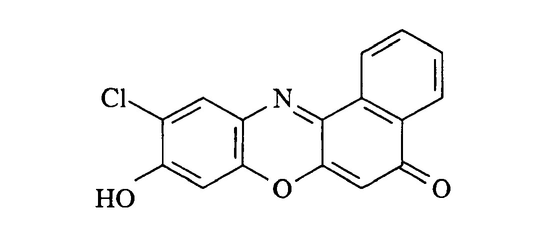

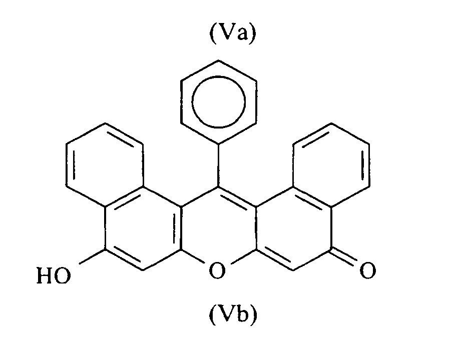

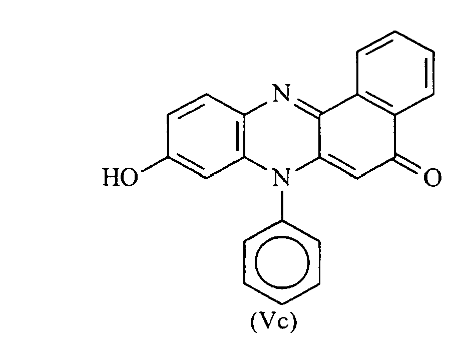

L'invention concerne également une composition pour la détection et/ou l'identification et/ou la quantification d'au moins un micro-organisme comprenant au moins un substrat enzymatique de formule générale (I) et un milieu réactionnel dudit micro-organismes ou desdits micro-organismes.

Par milieu réactionnel selon l'invention, on entend un milieu permettant le développement d'au moins une activité enzymatique d'au moins un micro-organisme, tel qu'un milieu de culture.

Le milieu réactionnel est solide, semi-solide ou liquide. Par milieu solide on entend par exemple un milieu gélosé. L'agar est le milieu traditionnel solide en microbiologie pour la culture des micro-organismes, mais il est possible d'utiliser de la gélatine ou de l'agarose. Un certain nombre de préparations sont disponibles comme l'agar Columbia, la gélose Trypcase-soja, la gélose Mac Conkey, la gélose Sabouraud, celles décrites dans le " Livret technique MILIEUX DE CULTURE " de la demanderesse ou plus généralement celles décrites dans le Handbook of Microbiological Media (CRC Press).

Avantageusement, le milieu réactionnel est un milieu gélifié contenant entre 2 et 40g/l préférentiellement entre 9 et 25 g/l d'agar.

Les substrats de la présente invention sont utilisables dans une large gamme de pH, notamment entre pH 5,5 et 10,0 et avantageusement entre pH 6,0 et 8,5.

Le milieu réactionnel peut aussi comprendre un ou plusieurs éléments en combinaison comme des acides aminés, des peptones, des hydrates de carbone, des nucléotides, des minéraux, des vitamines, des antibiotiques, des tensioactifs, des tampons, des sels de phosphate, d'ammonium, de sodium, de métaux. Des exemples de milieux sont décrits dans les brevets suivants de la demanderesse EP 0656421 ou WO 99/09207.

Dans un autre aspect de la présente invention, le milieu réactionnel comprend au moins deux substrats enzymatiques : un premier substrat enzymatique de la famille de l'invention et au moins un autre substrat enzymatique de la famille de l'invention et/ou un autre substrat enzymatique d'une autre famille de substrat. L'hydrolyse enzymatique du ou des autres substrats génère un signal détectable, différent du signal généré par le premier substrat, comme par exemple des produits colorés ou fluorescents différents, pour permettre la mise en évidence comme la détection et/ou l'identification et/ou la quantification d'un ou de plusieurs micro-organismes.

A titre d'exemple, on peut envisager les associations suivantes:
- un substrat de β-glucuronidase selon l'invention et X-β-D-glucoside (X est le 5-Bromo-4-chloro-3-indolyl-) pour un milieu pour les prélèvements urinaires (type CPS ID2, bioMérieux, Marcy l'Etoile, France) ;
- un substrat de β-galactosidase selon l'invention et X-β-D-glucoside pour un milieu pour les prélèvements urinaires (type CHROMagar Orientation (marque déposée) vendu par la société CHROMagar, Paris France ou Oxoid UTI vendu par la société OXOID, Hampshire, Angleterre) ;
- un substrat de β-glucuronidase selon l'invention et X-β-D-galactoside pour un milieu pour les *Escherichia coli* et les coliformes (type Coli ID, bioMérieux, Marcy l'Etoile, France) ;

A titre d'exemple, on peut utiliser un substrat d'hexosaminidase selon l'invention pour les levures et l'identification de *Candida albicans.*

Des substrats selon la présente invention peuvent notamment être introduits dans les milieux CPS ID2, SM ID, Albicans ID2, Coli ID, O157:H7 ID commercialisés par bioMérieux (Marcy l'Etoile, France) et comprenant tout ou partie de leur substrats enzymatiques, ainsi que dans les milieux comprenant de l'esculine tels que la gélose Bile Esculine avec ou sans agents sélectifs.

La concentration du substrat enzymatique dans le milieu réactionnel est comprise entre 0,02 et 1 g/l, avantageusement entre 0,03 et 0,30 g/l et préférentiellement entre 0,04 et 0,10 g/l.

La présente invention s'étend aussi à un kit de diagnostic comprenant une composition telle que définie précédemment et un contenant pour le milieu réactionnel. Par contenant, on entend tout support solide comme un flacon, un tube, une boite, une plaque de microtitration ou un consommable pour automate comme les galeries API ou les cartes VITEK (marques déposées, BioMérieux, Marcy l'Etoile, France).

Un autre aspect de la présente invention concerne un procédé pour la détection et/ou l'identification et/ou la quantification d'au moins un micro-organisme dans un échantillon où l'on met en contact le micro-organisme provenant de l'échantillon avec un milieu réactionnel contenant un substrat enzymatique de formule générale (I) et l'on détecte le produit coloré ou fluorescent formé par l'hydrolyse dudit substrat enzymatique.

Les substrats de formule générale (I) présentent l'avantage d'être utilisables indépendamment des conditions d'atmosphère d'incubation. La détection peut donc être mise en oeuvre par incubation du milieu réactionnel contenant le substrat enzymatique et inoculé par un micro-organisme au moins, dans une atmosphère contrôlée comme en condition aérobie, anaérobie, microaérophilie ou sous atmosphère CO₂ et de préférence aérobie, microaérophilie ou sous atmosphère CO₂.

L'échantillon à analyser est un échantillon clinique comme un prélèvement de salive, de sang, d'urine, des selles ou tout autre échantillon dont l'analyse peut aider un clinicien à poser un diagnostic. L'échantillon peut aussi être un échantillon de produit issu ou de base de l'industrie alimentaire et/ou pharmaceutique dans lequel il faut soit, garantir l'absence de micro-organisme pathogène, soit dénombrer une flore contaminante ou détecter des micro-organismes spécifiques. L'échantillon peut être mis en culture soit directement sur un milieu contenant un substrat de formule générale (I) soit après une étape de préculture par exemple dans le cas d'un échantillon alimentaire.

Le ou les micro-organismes identifiables, détectables ou quantifiables dans la présente invention sont des bactéries, des levures, des champignons, appartenant notamment aux groupes ou taxa suivant : *Enterobacteriaceae, Pseudomonadaceae, Nesseriaceae, Vibrionaceae, Pasteurellaceae, Campylobacter, Micrococcaceae, Streptococcaceae, Bacillus, Lactobacillus, Listeria, Corynebacterium, Gardnerella, Nocardia, Candida, Cryptococcus, Aspergillus* et plus particulièrement *Escherichia coli, E. coli* 0157:H7, *Shigella, Salmonella, Proteeae, Pseudomonas aeruginosa, Neisseria meningitidis, Enterococcus, Staphylococcus aureus, Bacillus cereus, Listeria monocytogenes, Streptococcus pyogenes, Streptococcus agalactiae,* des levures comme *Candida albicans, Candida glabrata, Candida tropicalis, Cryptococcus neoformans* et *Aspergillus fumigatus.* Ces micro-organismes peuvent être aérobies, aéroanaérobies, microaérophiles ou anaérobies.

Dans un autre aspect de la présente invention, les substrats enzymatiques sont utilisés dans des réactions de détection d'un analyte où une activité enzymatique (notamment la phosphatase alcaline ou la β-galactosidase) est mise en jeu comme : les réactions de détection d'anticorps ou d'antigène, ou d'acide nucléique, avec un format type ELISA (voir par exemple " les immunodosages de la théorie à la pratique " coordonné par Barbier Y., les éditions de l'ACOMEN, Lyon, p 109-133, 1988) ; dans des techniques de biologie moléculaire pour la mise en évidence d'un gène de type β-galactosidase (voir par exemple " Molecular cloning a laboratory manual ", 2nd ed., Sambrook, Fritsch, Maniatis, Cold Spring Harbor Laboratoty Press, section 16.56 et section 1.85, 1989) ; pour la détection d'acides nucléiques (voir par exemple " DNA probes ", 2nd edition, Keller G.H., Manak, M.M., Stockton press, section 5 à 9, 1993) ou dans des techniques histochimiques, cytochimiques ou de cytométrie de flux.

Les exemples qui suivent permettent d'illustrer quelques avantages de l'invention

### Exemple 1 : synthèse du p-naphtolbenzein-β-D-galactoside, PNB-gal produit (1)

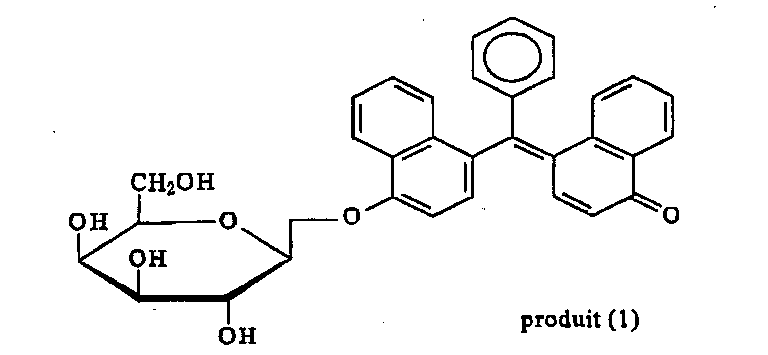

Le p-naphtolbenzein est obtenu chez Acros Organics (Geel, Belgique). Les autres réactifs sont obtenus chez Sigma Aldrich Chimie (St Quentin Fallavier, France). Le p-naphtolbenzein (1,87 g, 5 mmoles) est dissout dans 20 ml d'acétone sous une agitation vigoureuse. 5 ml d'hydroxyde de potassium à une concentration de 1,4 moles/litre sont ajoutés à cette solution suivie de 10 ml d'acétone. 5 ml d'eau sont alors additionnés goutte à goutte pour générer une solution bleu foncé. 10 mmoles d'α-acétobromogalactose (4,1g) dans 10ml d'acétone sont ajoutés à la solution. Pour maintenir le pH à une valeur supérieure à 11, 2 ml d'une solution d'hydroxyde de potassium à 20 moles/litre sont additionnés une première fois après 30 min d'agitation et une deuxième fois après 90 min d'agitation. Une troisième addition de solution alcaline est effectuée après 3,5 heures suivie d'une addition de 5 ml d'α-acétobromogalactose dans l'acétone à la même concentration que précédemment. Après 4,5 heures une dernière addition de solution alcaline a lieu et la solution est laissée sous agitation pendant la nuit.

L'acétone est éliminée sous pression réduite et la solution résiduelle est versée dans 300ml d'une solution de carbonate de sodium à 0,06mole/l à une température de 0°C sous agitation. Le précipité marron est filtré sous vide, lavé à l'eau et séché à l'air. Le solide est dissout dans 100ml de dichlorométhane et lavé intensément avec une solution d'hydroxyde de potassium à 0°C pour enlever le p-naphtolbenzein en excès. Le p-naphtolbenzein résiduel est éliminé par agitation sur une résine Dowex Marathon dans 100ml d'eau à pH 11 pendant 2 à 3 heures. Cette purification est suivie par chromatographie couche mince en utilisant un solvant acétate d'éthyle/toluène (3 :1) avec une révélation par l'ammoniaque. La solution jaune foncée est séchée la nuit sur sulfate de magnésium anhydre, évaporée sous pression réduite, reconstituée avec du méthanol puis évaporée à nouveau pour obtenir une mousse. Cette mousse est dissoute dans 50ml de méthanol et le produit est déprotégé pendant 5 heures en utilisant 20ml de méthoxyde de sodium dans le méthanol à 0,4 mole/l. La solution est alors ajustée à pH 6,5 en utilisant une résine IR120 (H⁺), séparée par décantation et le solvant est éliminé sous pression réduite. Le glycoside formé, p-naphtolbenzein-β-D-galactoside, (1,5g) se présente sous la forme d'une poudre jaune marron.

### Exemple 2 : Synthèse du dérivé acétate du p-naphtolbenzein.

Le p-naphtolbenzein est dissout dans la pyridine anhydre puis refroidi à 0°C et l'on additionne à cette solution de l'anhydride acétique (excès molaire de 5 fois) dissout dans la pyridine à froid. Après 24 heures à température ambiante, la solution est traitée avec de l'eau froide ajoutée goutte à goutte sous agitation pour décomposer l'excès d'agent acétylant. Un précipité de l'ester se forme qui est éliminé par filtration sous vide, lavé avec de l'acide acétique et recristallisé à chaud dans l'acide acétique.

### Exemple 3 : Synthèse du dérivé sulfate sous forme de sel de potassium du p-naphtolbenzein.

A une solution froide de p-naphtolbenzein dans la pyridine anhydre est ajoutée sous agitation 1,2 excès molaire d'acide chlorosulfonique à -15°C. Après 1 heure à -15°C et 30 minutes à température ambiante, l'excès de pyridine est évaporé sous pression réduite et le sel pyridinium est décomposé par dissolution dans l'éthanol et traitement avec une solution d'hydroxyde de potassium dans l'éthanol jusqu'à pH 10. Le sel de potassium est filtré sous vide, lavé avec de l'éthanol puis de l'éther diéthylique.

### Exemple 4: synthèse de la 8-chloro-1,2-benzoresorufin-β-D-glucoside, (CBR-glu) produit (2).

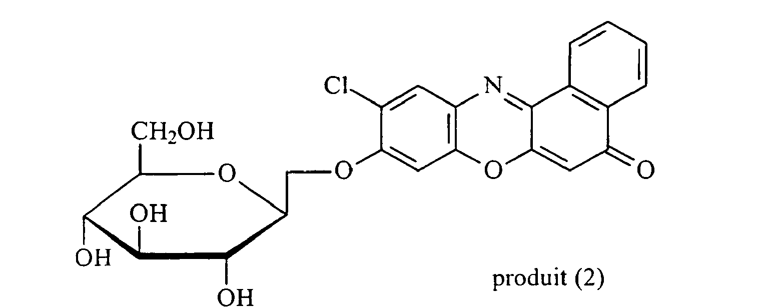

Le 6-nitroso-4-chlororesorcinol (2,94g, 20 mmoles) et le 1,3-dihydroxynaphthalène (3,20g, 20 mmoles) sont dissous séparément dans le butanol-1 (30ml) et mélangés. Le mélange réactionnel est chauffé entre 50 et 60°C à l'aide d'un bain marie et on additionne de l'acide sulfurique (1,0g) goutte à goutte sous agitation pendant 30 secondes. La solution devient rouge foncée et des cristaux se forment rapidement. Après 15 min à 50°C et 1 heure à température ambiante, le produit est filtré sous vide et recristallisé à partir de butanol-1 chaud pour donner 2,8g de cristaux rouges foncés brillants de 8-chloro-1,2-benzoresorufin.

Le dérivé glucoside (2) est préparé par une réaction de Koenigs-Knorr comme décrit précédemment pour le dérivé p-naphtolbenzein. Le glucoside sous une forme protégée tétraacétylée est isolé par évaporation rotative à partir de dichlorométhane et déprotégé directement en utilisant une quantité catalytique de méthoxyde de sodium dans le méthanol anhydre. Le glycoside se présente sous la forme d'une poudre jaune orangée.

### Exemple 5 : synthèse de la 8-chloro-1,2-benzoresorufin-β-D-galactoside (CBR-gal) produit (3)

Le mode de préparation du dérivé β-D-galactoside (3) est identique à celui décrit dans l'exemple 4 en utilisant le dérivé β-D-galactoside sous forme protégée tétraacétylée à la place de l'équivalent glucoside.

### Exemple 6: synthèse de la 3-hydroxy-9-phényl-1,2:7,8-dibenzo-6-fluorone-β-D-galactoside produit (4).

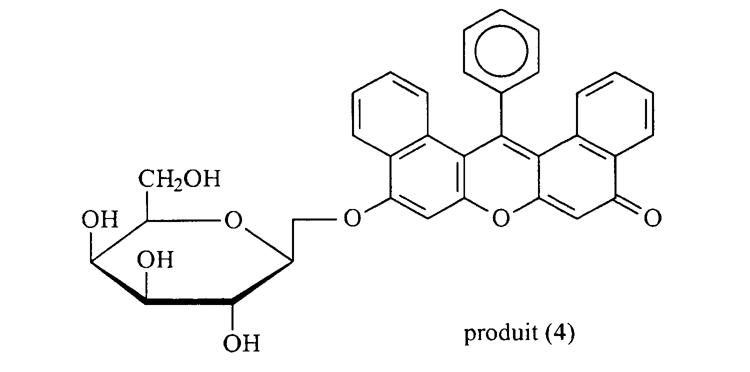

La 3-hydroxy-9-phényl-1,2 :7,8-dibenzo-6-fluorone est préparé à partir du 1,3-dihydroxynaphtalène (Aldrich, 14529-7) par condensation à chaud avec le α,α,α-trifluorotoluène (Aldrich, T6370-3) en présence d'un acide de Lewis comme SnCl₄ ou ZnCl₂ dans un solvant à haut point d'ébullition comme le chlorobenzène ou le xylène. Après hydrolyse de l'acide de Lewis, et purification du composé intermédiaire, le dérivé galactoside (4) est préparé comme décrit dans l'exemple 1.

### Exemple 6 bis : Synthèse du naphtosafranol (9-hydroxy-7phenol-5benzo[a]phenazinone) :

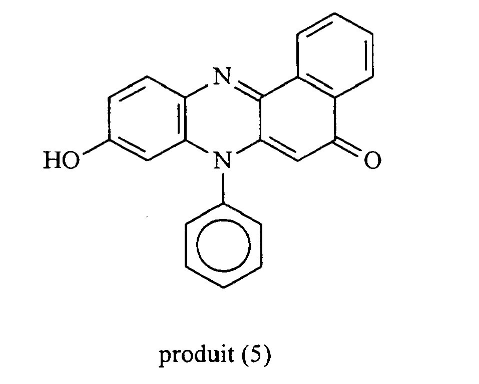

Un mélange de 50 g d'acide sulfurique et 130 ml d'eau sont additionnés lentement dans une solution, maintenue à 0°C et sous agitation, de phénol (20g), nitrite de sodium(18g) et d'hydroxyde de sodium (9g) dans 500 ml d'eau. Après 2 heures de réaction, le produit est collecté, lavé à l'eau glacée. Après recristallisation dans l'eau, le produit p-nitrosophénol est isolé pur (13,4g).

Le p-nitrosophénol ainsi préparé (12,3 g, 0,1 mole) ainsi que de la N-phényl-2-naphtylamine (14,5g, 0,067 mole, Aldrich, 17,805-5) sont dissous dans l'éthanol (400ml) et le mélange est refroidi à 15°C. A cette solution, l'on ajoute de l'HCl concentré (15g). La température remonte spontanément et le produit isorosindone (sous forme de sel chlorhydrique) est séparé par filtration et recristallisé dans un mélange éthanol-eau (50%-50%) Le produit recristallisé peut être converti dans sa forme base libre en le dissolvant dans un premier temps dans l'éthanol puis addition d'hydroxyde d'ammonium et enfin ajout de la solution dans l'eau chaude.
La base libre (isorosindone) précipite sous forme de cristaux marron foncé en refroidissant la solution. Une filtration sous vide conduit à 15 g de produit pur.

Le produit cible (5) est obtenu en chauffant l'isorosindone à reflux dans une solution concentré de KOH dans le butanol-1. Le produit est purifié par chromatographie flash sur silice avec l'éthylacétate comme éluant.

### Exemple 6 ter : Le dérivé galactoside du naphtosafranol est préparé selon le même protocole que celui décrit pour l'exemple 1.

### Exemple 7 : utilisation du p-naphtolbenzein-β-D-galactoside (PNB-gal) pour la détection de microorganismes possédant une activité β-galactosidase.

Un milieu solide agar comprenant le PNB-gal est préparé comme suit : 41g d'agar Columbia sont additionnés à 1 litre d'eau distillée avec 100mg de PNB-gal et 30 mg d'IPTG (isopropyl-β-D-thiogalactoside) pour faciliter l'induction de l'activité β-galactosidase. L'agar est stérilisé par autoclavage à 116°C pendant 10 min. Le milieu est refroidi lentement à 55°C avant d'être réparti dans des boites de 20ml.

367 souches différentes incluant 303 *Enterobacteriaceae* sont collectées à partir d'échantillons cliniques et environnementaux et identifiés par la galerie API 20E (BioMérieux, France) comme méthode de référence. Les souches sont cultivées sur un milieu Columbia agar à 37°C pendant 24 heures puis un inoculum d'environ 10⁸ organismes /ml (équivalent à un standard McFarland de 0,5) est réalisé pour chaque souche. En utilisant un inoculateur Denley, 1 microlitre de chaque suspension est inoculé dans le milieu PNB-gal préparé ci-dessus à raison de 20 souches par boite. Toutes les boites sont incubées à 37°C pendant 18 heures. Après incubation, les boites sont examinées en fonction de la présence d'une colonie violette en comparaison avec la croissance sur le milieu Columbia agar. Les résultats sont présentés dans le tableau 1.

**Tableau l**

| Espèces gram moins | Nombre de souches testées pour l'espèce | Résultats positifs avec le substrat PNB-gal (%) |
|---|---|---|
| *Acinetobacter spp.* | 53 | 0 |
| *Aeromonas caviae* | 7 | 86 |
| *Aeromonas hydrophila* | 3 | 100 |
| *Citrobacter diversus* | 9 | 89 |
| *Citrobacter freundii* | 16 | 100 |
| *Enterobacter aerogenes* | 9 | 100 |
| *Enterobacter agglomerans* | 1 | 100 |
| *Enterobacter cloacae* | 21 | 100 |
| *Escherichia coli* | 41 | 95 |
| *Escherichia hermannii* | 1 | 100 |
| *Hafnia alvei* | 10 | 30 |
| *Klebsiella oxytoca* | 13 | 100 |
| *Klebsiella ozaenae* | 3 | 67 |
| *Klebsiella pneumoniae* | 19 | 100 |
| *Morganella morganii* | 12 | 0 |
| *Proteus mirabilis* | 16 | 0 |
| *Proteus penneri* | 1 | 0 |
| *Proteus vulgaris* | 4 | 0 |
| *Providencia alcalifaciens* | 3 | 0 |
| *Providencia rettgeri* | 3 | 0 |
| *Providencia stuartii* | 10 | 0 |
| *Salmonella spp.* | 64 | 0 |
| *Serratia odorifera* | 1 | 100 |
| *Serratia spp.* | 14 | 79 |
| *Shigella boydii* | 1 | 0 |
| *Shigella dysenteriae* | 2 | 0 |
| *Shigella flexneri* | 2 | 0 |
| *Shigella sonnei* | 10 | 100 |
| *Vibrio cholerae* | 1 | 100 |
| *Yersinia enterocolitica* | 14 | 7 |
| *Yersinia pseudotuberculosis* | 3 | 0 |

Ce tableau montre une bonne efficacité du substrat comme indicateur de l'activité β-galactosidase. Aucune souche sans l'activité β-galactosidase n'est détectée ce qui signifie qu'il n'y a pas de faux positifs et la sensibilité sur les souches *Enterobacteriaceae* est de 95,1% par rapport à la méthode de référence API 20E.

### Exemple 8 :Influence du pH sur la révélation de la β-D-galactosidase en présence de p-naphtolbenzein-β-D-galactoside PNB-gal.

Le milieu ci-après, base Columbia à une concentration de 46,37g/l, isopropyl-thio-β-D-galactoside (IPTG) à 0,03g/l, p-naphtolbenzein-β-D-galactoside à 0,1 g/l, a été ajusté à différents pH (5,5 - 6,0 - 6,5 - 7,0 - 7,5 - 8,0 - 8,5) à 24°C après autoclavage des milieux. Ces différents milieux répartis en boîtes de Pétri ont été inoculé en isolement en trois cadrans à partir de suspension à 0,5 McFarland de micro-organismes bien caractérisés issus de collection type ATCC ou NCTC. Les boîtes ont été incubées à 37°C pendant 48 heures. Les colonies formées ont été examinées visuellement après 24 et 48 heures d'incubation, la couleur a été notée. Les résultats sont présentés dans le tableau II ci-après :

**Tableau II**

| Souches testées | TI | pH 5,7 | pH 6,0 | PH 6,5 | pH 7,0 | pH 7,5 | pH 8,0 | pH 8,5 |
|---|---|---|---|---|---|---|---|---|
| *E. coli* | 24 H | - | - | - | - | - | - | - |
| *032* | 48 H | - | - | - | - | - | - | - |
| *E. coli* | 24 H | Orange | Orange | Orange | Orange | Orange | Orange | Marron |
| *115* | 48 H | Orange | Orange | Orange | Orange | Brun | Brun | Brun |
| *S*. *marcescens* | 24 H | Marron | Marron | Marron | Marron | Marron | Vert | Vert |
| *217* | 48 H | Marron | Marron | Marron | Marron | Vert | Kaki | Kaki |
| *E. cloacae* | 24 H | Orange | Orange | Orange | Orange | Brun | Brun | Brun |
| *061* | 48 H | Marron | Marron | Marron | Vert | Vert | Kaki | Kaki |
| *S*. *typhimurium* | 24 H | - | - | - | - | - | - | - |
| *010* | 48 H | - | - | - | - | - | - | - |
| *E. faecalis* | 24 H | - | - | - | - | - | - | - |
| *117* | 48 H | Orange | Orange | Orange | Orange | Orange | Orange | Orange |
| *S. agalactiae* | 24 H | - | - | - | - | - | - | - |
| *015* | 48H | - | - | - | - | - | - | - |
| *S*. *aureus* | 24 H | - | - | - | - | - | - | - |
| *008* | 48 H | - | - | - | - | - | - | - |

| | | | | | | | | |
|---|---|---|---|---|---|---|---|---|
| - signifie une absence de coloration, TI représente le temps d'incubation sur les boites | | | | | | | | |

Suivant les souches et la durée d'incubation, la couleur des colonies varie en fonction du pH. En général, elle est plus orange à brune à pH acide et vert à kaki à pH alcalin. Ceci peut permettre de différentier différents groupes de micro-organismes, d'adapter la couleur en fonction des besoins (association avec d'autres substrats enzymatiques, indicateurs de pH) ou de mettre en évidence plusieurs métabolismes (hydrolyse enzymatique et variation de pH) ce qui est un avantage du substrat PNB-gal.

### Exemple 9: Influence du milieu réactionnel sur la révélation de la β-D-galactosidase en présence de p-naphtolbenzein-β-D-galactoside, PNB-gal.

Dans les milieux Columbia , Trypcase Soja (GTS) et MacConkey Sorbitol (SMAC), il a été ajouté le mélange suivant:

| | |
|---|---|
| IsoPropyl-Thio-β-D-galactoside | 0,03 g/l |
| p-naphtolbenzein-β-D-galactoside | 0,1g/l |

Après autoclavage, le pH des milieux était à 23°C : 7,1 - 7,1 et 7,2 sur les milieux SAMC, GTS et Columbia respectivement. Ces différents milieux répartis en boîtes de Pétri ont été inoculé en isolement en trois cadrans à partir de suspension à 0,5 McFarland de micro-organismes bien caractérisés issus de collection type ATCC ou NCTC. Les boîtes ont été incubées à 37°C pendant 48 heures. Les colonies formées ont été examinées visuellement après 24 et 48 heures d'incubation, la couleur a été notée. Les résultats sont présentés dans le tableau III ci-après :

**Tableau III**

| Souches testées | TI | milieu SMAC | milieu GTS | milieu Columbia |
|---|---|---|---|---|
| *E. coli* | 24 H | - | - | - |
| *032* | 48 H | - | - | - |
| *E. coli* | 24 H | Orange | Orange | Brun-Orange |
| *115* | 48 H | Orange | Orange | Brun |
| *E. coli* | 24 H | Orange | Brun-Orange | Brun |
| *206* | 48 H | Orange | Brun-Orange | Brun |
| *S. marcescens* | 24 H | Fuchsia | Brun-Orange | Brun |
| *217* | 48 H | Fuchsia | Brun | Brun |
| *E. cloacae* | 24 H | Orange | Brun-Orange | Brun |
| *061* | 48 H | Orange | Brun | Brun |
| *S. typhimurium* | 24 H | - | - | - |
| *010* | 48 H | - | - | - |
| *E. faecalis* | 24 H | - | - | - |
| *010* | 48 H | - | Orange | Orange |
| *S. agalactiae* | 24 H | - | - | - |
| *015* | 48 H | - | - | - |
| *S. aureus* | 24 H | - | - | - |
| *008* | 48 H | - | - | - |

| | | | | |
|---|---|---|---|---|
| - signifie une absence de coloration, TI représente le temps d'incubation sur boite. | | | | |

Suivant les souches et la durée d'incubation, la couleur des colonies varie en fonction du milieu. En général, elle est orange sur le milieu SMAC et brune sur le milieu Columbia, sans que cette différence de couleur ne puisse être lié au pH du milieu. Sur le milieu SMAC, il est possible de différentier *Serratia marcescens* des autres bactéries, ce qui n'est pas le cas sur les deux autres milieux. De plus, il peut être intéressant de pouvoir adapter la couleur des colonies obtenue avec le p-naphtolbenzein-β-D-galactoside en fonction de l'utilisation d'autres substrats enzymatiques donnant d'autres couleurs (rose ou brune par exemple).

### Exemple 10 : Influence de l'atmosphère d'incubation sur la révélation de la β-D-galactosidase en présence de p-naphtolbenzein-β-D-galactoside, PNB-gal.

Dans le milieu ci-après :

| | |
|---|---|
| Extrait de levure | 6g/l |
| Peptone de gélatine | 5g/l |
| NaCl | 8g/l |
| Carbonate de Na | 0,1g/l |
| IsoPropyl-Thio-β-D-galactoside | 0,03g/l |
| Agar | 13g/l |

est ajouté soit du 5-Bromo-4-chloro-3-indolyl-β-D-galactoside (X-Gal), soit du p-naphtolbenzein-β-D-galactoside (PNB-Gal) à 0,1 g/l. Nous avons inoculé ces différents milieux répartis en boîtes de Pétri avec des micro-organismes bien caractérisés issus de collection type ATCC ou NCTC. Les boîtes ont été incubées à 37°C pendant 48 heures sous différentes atmosphères : aérobiose (AE), microaérophilie (MAP), anaérobiose (ANA), CO₂ par l'intermédiaire du système GENbox (BioMérieux, France) pour le contrôle de l'atmosphère. Les colonies formées ont été examinées visuellement après 24 et 48 heures d'incubation, l'intensité de coloration a été notée. Les résultats sont présentés dans le tableau IV ci-après :

**Tableau IV**

| | | **X-Gal** | | | | **PNB-Gal** | | | |
|---|---|---|---|---|---|---|---|---|---|
| | | **AE** | **MAP** | **ANA** | **CO₂** | **AE** | **MAP** | **ANA** | **CO₂** |
| Souches | TI | Essai 1 | Essai 2 | Essai | 3 Essai 4 | Essai 5 | Essai 6 | Essai 7 | Essai 8 |
| *E. coli* | 24 H | 3* | 2 | - | 2 | 3 | 3 | 1 | 3 |
| *115* | 48 H | 3 | 2 | 1 | 2 | 3 | 3 | 3 | 3 |
| *E. coli* | 24 H | 3 | 1 | 1 | 3 | 3 | 3 | 2 | 3 |
| *206* | 48 H | 3 | 1 | 1 | 3 | 3 | 3 | 3 | 3 |
| *S. marcescen* | 24 H | - | - | - | - | - | - | - | - |
| *042* | 48 H | 1 | 1 | - | 2 | 3 | 1 | - | 3 |
| *E. cloacae* | 24 H | 2 | 1 | - | 2 | 2 | 2 | 1 | 2 |
| *061* | 48H | 2 | 1 | 1 | 2 | 2 | 2 | 3 | 2 |
| *P. vulgaris* | 24 H | - | - | - | - | - | - | - | - |
| *140* | 48 H | - | - | - | - | - | - | - | - |
| *E. faecalis* | 24 H | - | - | - | - | - | - | - | - |
| *117* | 48H | - | - | - | - | - | - | - | - |
| *E. faecium* | 24 H | 1 | - | - | - | - | - | - | - |
| *255* | 48 H | 3 | 1 | - | 1 | 3 | - | - | - |
| *S. aureus* | 24 H | - | - | - | - | - | - | - | - |
| *008* | 48 H | - | - | - | - | - | - | - | - |

| | | | | | | | | | |
|---|---|---|---|---|---|---|---|---|---|
| * : intensité de coloration (échelle arbitraire à partir d'une observation visuelle) ; - signifie une absence de coloration ; TI signifie temps d'incubation. | | | | | | | | | |

Si le X-Gal permet de détecter une activité sur la souche d*'E. faecium* contrairement au PNB-Gal, pour les souches qui hydrolysent fortement le X-Gal en aérobiose (*E. coli, E. cloacae),* le PNB-Gal permet de détecter cette activité β-D-galactosidase indépendamment de l'atmosphère et plus intensément. En effet, si les intensités sont faibles voire très faibles dans les conditions de l'essai 3, elles sont fortes dans les conditions de l'essai 7.

### Exemple 11 : utilisation du 8-chloro-1,2-benzoresorufin-β-D-galactoside (CBR-gal) pour la détection de micro-organismes possédant une activité β-galactosidase dans un milieu solide.

Le substrat CBR-gal est incorporé dans un agar Columbia aux concentration suivantes 2, 6, 10 et 20 mg/100ml et le milieu ainsi préparé est réparti dans des boites. Chaque boite est inoculée avec les organismes suivants *E*. *coli* (NCTC, 10418), *K. pneumoniae* (NCTC, 10896), *P. rettgeri* (NCTC, 7475), *E. cloacae* (NCTC, 11936), *S. marcescens* (NCTC, 10211) et *S*. *typhimurium* (NCTC, 74). De l'IPTG est incorporé dans tous ces milieux à une concentration de 3 mg pour 100 ml d'agar Columbia. Toutes les boites sont incubées à 37°C pendant la nuit.

Les souches positives pour l'activité β-galactosidase *(E. coli, K. pneumoniae, E. cloacae* et *S*. *marcescens*) hydrolysent rapidement le substrat après une incubation d'une nuit en donnant une coloration rose qui est limitée à la colonie bactérienne. Toutes les concentrations testées permettent de différencier les espèces mais la concentration optimale pour avoir une coloration rose clairement visible sans bruit de fond se situe vers 10mg/100ml. Aucune inhibition de croissance n'est visible pour toutes les concentrations testées.

### Exemple 12 : utilisation du 8 chloro-1,2 benzoresorufin-β D galactoside (CBR-gal) pour la détection de micro-organismes possédant une activité β galactosidase dans un milieu liquide.

Le substrat CBR-gal est testé dans un milieu liquide à différentes concentrations de 0,5mg/ml à 0,0078mg/ml. Le milieu réactionnel liquide dans lequel est incorporé le substrat est composé d'un tampon phosphate pH 7,4 contenant 0,5% de bouillon nutritif et 0,5% de NaCl. 30 microlitres /ml d'IPTG sont ajoutés dans le milieu pour toutes les concentrations de substrat. Les souches testées sont *E. coli* (NCTC, 10418), *K. pneumoniae* (NCTC, 10896), *P rettgeri* (NCTC, 7475), *E. cloacae* (NCTC, 11936), *S. marcescens* (NCTC, 10211) et *S*. *typhimurium* (NCTC, 74) avec un inoculum de 0,5 McFarland.

Toutes les souches possédant une activité β-galactosidase générent une coloration rose au cours du temps comme résumé dans le tableau V ci-après.

**Tableau V**

| | Concentration en substrat CBR-gal | | | | | | |
|---|---|---|---|---|---|---|---|
| Organismes | 0.5 mg/ml | 0.25 mg/ml | 0.125 mg/ml | 0.0625 mg/ml | 0.0313 mg/ml | 0.0156 mg/ml | 0.0078 mg/ml |
| *E. coli* | 2* | 2 | 4 | 4 | 24 | - | - |
| *K. pneumoniae* | 2 | 2 | 4 | 4 | 24 | - | - |
| *P. rettgeri* | - | - | - | - | - | - | - |
| *E. cloacae* | 2 | 2 | 4 | 4 | 24 | - | - |
| *S. marcescens* | 24 | 24 | 24 | 24 | 24 | - | - |
| *S. typhimurium* | - | - | - | - | - | - | - |

| | | | | | | | |
|---|---|---|---|---|---|---|---|
| * : les résultats sont donnés en heures. - signifie une absence de coloration même après 24 heures. | | | | | | | |

## Revendications

1. Substrat enzymatique de formule générale (I) : 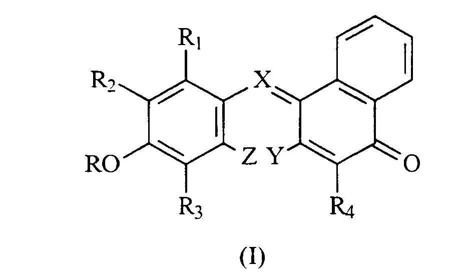 dans laquelle
X représente un atome d'azote ou un atome de carbone substitué par un groupement phényle, ledit groupement phényle étant éventuellement substitué en position méta ou para,
Y et Z représentent un atome d'hydrogène lorsque X représente un atome de carbone ou Y et Z représentent ensemble une liaison éther, thioéther ou amine substituée éventuellement par un groupement alkyle ou aryle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non,
R₃ et R₄ représentent chacun indépendamment l'un de l'autre H, Cl, F, I, Br et peuvent être identiques ou différents,
R représente un groupement tel que la liaison O-R est hydrolysable par une enzyme.

2. Substrat enzymatique selon la revendication 1 **caractérisé par le fait que** l'enzyme est de la famille des osidases comme la β-glucuronidase, la β-galactosidase, β-glucosidase, la 6-phosphogalactohydrolase, l'α-galactosidase, l'α-amylase, l'α-glucosidase, les hexosaminidases comme la N-acétyl-β-glucosaminidase ou la N-acétyl-β-galactosaminidase, de la famille des estérases, des lipases, des phosphatases, des sulfatases, des DNases, des peptidases et des protéases.

3. Substrat enzymatique selon l'une quelconque des revendications 1 ou 2 **caractérisé par le fait que** R est un résidu de type sucre α- ou β- préférentiellement le β-D-glucose ou le β-D-galactose ou un β-D-glucuronate ou un phosphate, un sulfate, un acétate.

4. Substrat enzymatique selon l'une quelconque des revendications 1 à 3 de formule générale (II) : 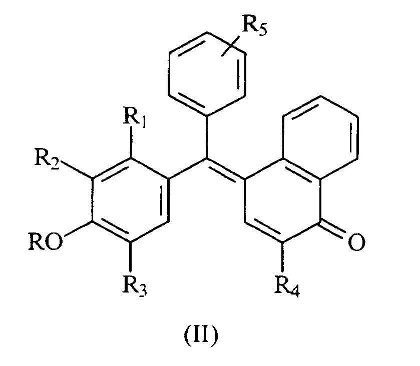 dans laquelle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non,
R₃ et R₄ représentent chacun indépendamment l'un de l'autre H, Cl, F, I, Br et peuvent être identiques ou différents,
R₅ représente H, NO₂, CF₃, CN, OCH₃, F, I, Cl, Br, SO₃H, CO₂H en position méta ou para,
R représente un groupement tel que la liaison O-R est hydrolysable par une enzyme.

5. Substrat enzymatique selon le revendication 4 **caractérisé par le fait que**
R₁ et R₂ ensemble représentent un noyau benzénique fusionné,
R₃ et R₄ représentent un atome d'hydrogène,
R₅ représente un hydrogène,
R représente un résidu de type sucre α- ou β- préférentiellement le β-D-glucose ou le β-D-galactose, ou un sulfate, un acétate.

6. Substrat enzymatique selon l'une quelconque des revendications 1 à 3 de formule générale (III) 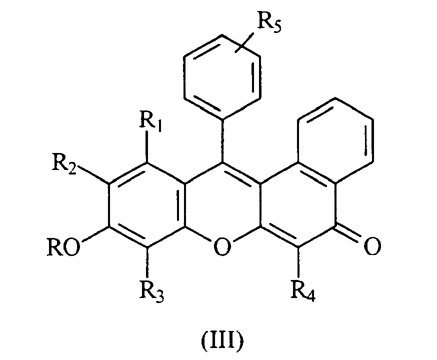 dans laquelle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non,
R₃ et R₄ représentent chacun indépendamment l'un de l'autre H, Cl, F, I, Br et peuvent être identiques ou différents,
R₅ représente H, NO₂, CF₃, CN, OCH₃, F, I, Cl, Br, SO₃H, CO₂H en position méta ou para.
R représente un groupement tel que la liaison O-R est hydrolysable par une enzyme.

7. Substrat enzymatique selon la revendication 6 **caractérisé par le fait que**
R₁ et R₂ ensemble représentent un noyau benzénique fusionné,
R₃ et R₄ représentent un atome d'hydrogène,
R₅ représente H
R représente un résidu de type sucre α- ou β- préférentiellement le β-D-glucose ou le β-D-galactose.

8. Substrat enzymatique selon l'une quelconque des revendications 1 à 3 de formule générale (IVa) : 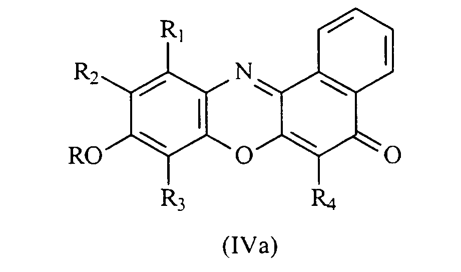 dans laquelle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non. Préférentiellement R₁ représente H et R₂ représente Cl.
R₃ et R₄ représentent chacun indépendamment l'un de l'autre H, Cl, F, I, Br et peuvent être identiques ou différents. Préférentiellement R₃ et R₄ représentent H.
R représente un groupement tel que la liaison O-R est hydrolysable par une enzyme. En particulier, R représente un résidu de type sucre α- ou β-, préférentiellement le β-D-glucose ou le β-D-galactose.

9. Substrat enzymatique selon l'une quelconque des revendications 1 à 3 de formule générale (IVb) : 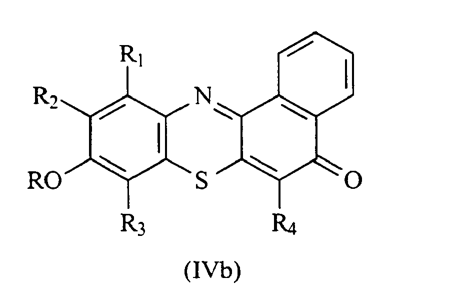 dans laquelle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non. Préférentiellement R₁ représente H et R₂ représente Cl.
R₃ et R₄ représentent chacun indépendamment l'un de l'autre H, Cl, F, I, Br et peuvent être identiques ou différents. Préférentiellement R₃ et R₄ représentent H.
R représente un groupement tel que la liaison O-R est hydrolysable par une enzyme. En particulier, R représente un résidu de type sucre α- ou β-, préférentiellement le β-D-glucose ou le β-D-galactose.

10. Substrat enzymatique selon l'une quelconque des revendications 1 à 3 de formule générale (IVc) : 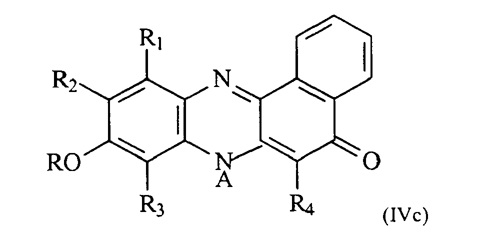 dans laquelle,
A représente un groupe aryle ou alkyle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non. Préférentiellement R₁ représente H et R₂ représente Cl.
R₃ et R₄ représentent chacun indépendamment l'un de l'autre H, Cl, F, I, Br et peuvent être identiques ou différents. Préférentiellement R₃ et R₄ représentent H.
R représente un groupement tel que la liaison O-R est hydrolysable par une enzyme. En particulier, R représente un résidu de type sucre α- ou β-, préférentiellement le β-D-glucose ou le β-D-galactose.

11. Substrat enzymatique selon la revendication 8 **caractérisé par le fait que**
R₁ représente H et R₂ représente Cl,
R₃ et R₄ représentent H,
R représente un résidu de type sucre α- ou β- préférentiellement le β-D-glucose ou le β-D-galactose.

12. Substrat enzymatique selon la revendication 10 **caractérisé par le fait que**
R₁, R₃ et R₄ représentent H,
A représente un phényle,
R représente un résidu de type sucre α- ou β- préférentiellement le β-D-glucose ou le β-D-galactose.

13. Procédé de synthèse d'un substrat enzymatique selon la revendication 1 **caractérisé par le fait que** l'on prépare un composé intermédiaire de formule générale (V) 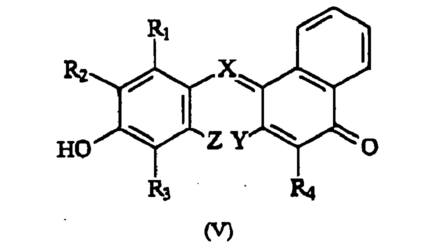 dans laquelle
X représente un atome d'azote ou un atome de carbone substitué par un groupement phényle, ledit groupement phényle étant éventuellement substitué en position méta ou para,
Y et Z représentent un atome d'hydrogène lorsque X représente un atome de carbone ou Y et Z représentent ensemble une liaison éther, thioéther ou amine substituée éventuellement par un groupement alkyle ou aryle,
R₁ et R₂ représentent chacun indépendamment l'un de l'autre H, Cl, F, I, Br et peuvent être identiques ou différents ou R₁ et R₂ ensemble représentent un noyau benzénique fusionné substitué ou non,
R₃ et R₄ représentent chacun indépendamment l'un de l'autre H, Cl, F, I, Br et peuvent être identiques ou différents, et l'on greffe sur l'hydroxyle un groupement R convenablement protégé.

14. Procédé selon la revendication 13, **caractérisé par le fait que** le composé intermédiaire répond à la formule (Va) suivante

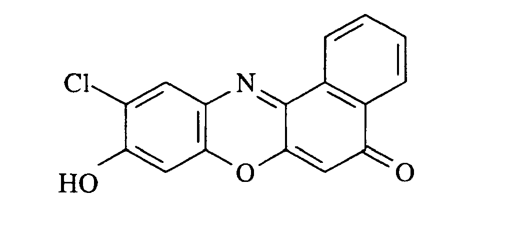

15. Procédé selon la revendication 13, **caractérisé par le fait que** le composé intermédiaire répond à la formule (Vb) suivante

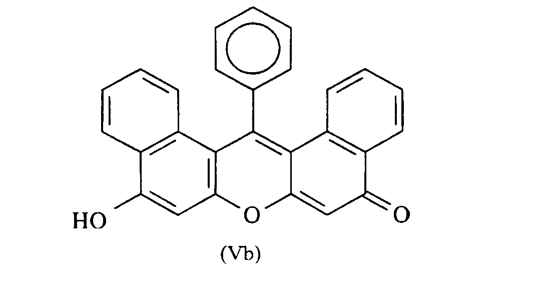

16. Procédé selon la revendication 13, **caractérisé par le fait que** le composé intermédiaire répond à la formule (Vc) suivante

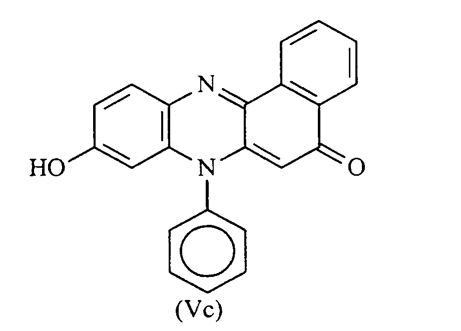

17. Composition pour la détection et/ou l'identification et/ou la quantification d'au moins un micro-organisme comprenant au moins un substrat enzymatique selon l'une quelconque des revendications 1 à 12 et un milieu réactionnel approprié audit micro-organisme ou auxdits micro-organismes.

18. Composition selon la revendication 17, **caractérisée par le fait que** ladite composition comporte au moins deux substrats selon l'une quelconque des revendications 1 à 12, et dont l'hydrolyse enzymatique conduit à des produits colorés et/ou fluorescents différents.

19. Composition selon la revendication 17 ou 18, **caractérisée par le fait qu'**elle comprend en outre un autre substrat enzymatique différent des substrats selon l'une quelconque des revendications 1 à 12, et dont l'hydrolyse enzymatique conduit à un produit coloré ou fluorescent différent de celui libéré par l'hydrolyse enzymatique du substrat utilisé selon la revendication 17 ou de ceux libérés par l'hydrolyse enzymatique des substrats utilisés selon la revendications 18.

20. Composition selon la revendication 17, 18 ou 19, **caractérisée par le fait que** le milieu réactionnel est solide, semi solide ou liquide.

21. Composition selon l'une quelconque des revendications 17 à 20, **caractérisée par le fait que** la concentration du substrat enzymatique dans le milieu réactionnel est comprise entre 0,02 et 1 g/l, avantageusement entre 0,03 et 0,30 g/l et préférentiellement entre 0,04 et 0,10 g/l.

22. Kit de diagnostic comprenant une composition selon l'une quelconque des revendications 17 à 21 et un contenant pour le milieu réactionnel.

23. Procédé pour la détection et/ou l'identification et/ou la quantification d'au moins un micro-organisme dans un échantillon, **caractérisé par le fait que** :
l'on met en contact le micro-organisme provenant de l'échantillon avec un milieu réactionnel contenant un substrat enzymatique tel que décrit dans les revendications 1 à 12 pour obtenir un milieu inoculé, et l'on détecte le produit coloré ou fluorescent formé par l'hydrolyse dudit substrat enzymatique.

24. Procédé selon la revendication 23, **caractérisé par le fait que** l'on incube le milieu réactionnel inoculé dans une atmosphère contrôlée comme en condition aérobie, anaérobie, microaérophilie ou sous atmosphère CO₂, préférentiellement dans des conditions aérobie, microaérophilie et CO₂

25. Utilisation d'un substrat enzymatique selon l'une quelconque des revendications 1 à 12, pour la détection d'un analyte dans des techniques mettant en jeu une activité enzymatique telles que la technique ELISA où l'analyte est un anticorps ou un antigène ou un acide nucléique, ou des techniques de biologie moléculaire où l'analyte est un gène de type β-galactosidase.

## Claims

1. Enzymatic substrate of general formula (I): 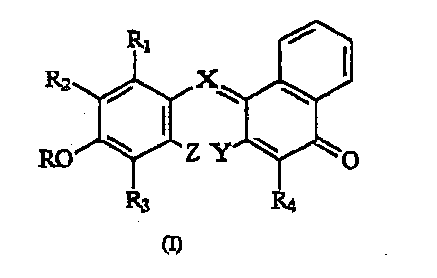 in which
X represents a nitrogen atom or a carbon atom substituted with a phenyl group, said phenyl group optionally being substituted in the meta or para position,
Y and Z represent a hydrogen atom when X represents a carbon atom, or Y and Z together represent an ether, thioether or amine bond optionally substituted with an alkyl or aryl group,
R₁ and R₂ each represent, independently of one another, H, Cl, F, I or Br, and may be identical or different, or R₁ and R₂ together represent a fused benzene ring which may or may not be substituted,
R₃ and R₄ each represent, independently of one another, H, Cl, F, I or Br and may be identical or different,
R represents a group such that the O-R bond can be hydrolysed by an enzyme.

2. Enzymatic substrate according to Claim 1, **characterized in that** the enzyme is from the family of osidases, such as β-glucuronidase, β-galactosidase, β-glucosidase, 6-phosphogalactohydrolase, α-galactosidase, α-amylase, α-glucosidase or the hexosaminidases, for instance N-acetyl-β-glucosaminidase or N-acetyl-β-galactosaminidase, or from the family of esterases, of lipases, of phosphatases, of sulfatases, of DNAases, of peptidases and of proteases.

3. Enzymatic substrate according to either of Claims 1 and 2, **characterized in that** R is a residue of the α- or β-sugar type, preferentially β-D-glucose or β-D-galactose or a β-D-glucuronate, or a phosphate, a sulfate or an acetate.

4. Enzymatic substrate according to any one of Claims 1 to 3, of general formula (II) : 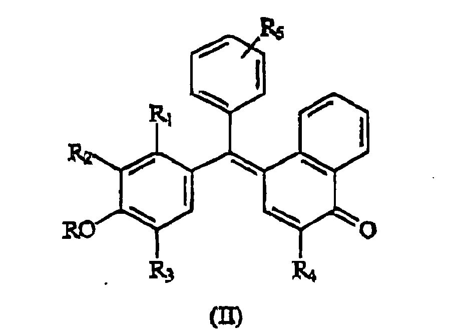 in which,
R₁ and R₂ each represent, independently of one another, H, Cl, F, I or Br, and may be identical or different, or R₁ and R₂ together represent a fused benzene ring which may or may not be substituted,
R₃ and R₄ each represent, independently of one another, H, Cl, F, I or Br, and may be identical or different,
R₅ represents H, NO₂, CF₃, CN, OCH₃, F, I, Cl, Br, SO₃H or CO₂H in the meta or para position,
R represents a group such that the O-R bond can be hydrolysed by an enzyme.

5. Enzymatic substrate according to Claim 4, **characterized in that**
R₁ and R₂ together represent a fused benzene ring,
R₃ and R₄ represent a hydrogen atom,
R₅ represents a hydrogen,
R represents a residue of the α- or β-sugar type, preferentially β-D-glucose or β-D-galactose, or a sulfate or an acetate.

6. Enzymatic substrate according to any one of Claims 1 to 3, of general formula (III) 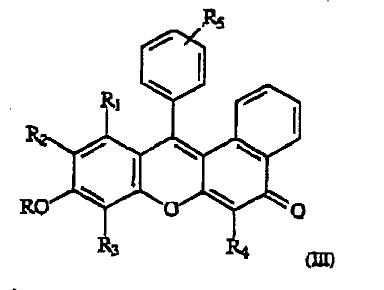 in which,
R₁ and R₂ each represent, independently of one another, H, Cl, F, I or Br, and may be identical or different, or R₁ and R₂ together represent a fused benzene ring which may or may not be substituted,
R₃ and R₄ each represent, independently of one another, H, Cl, F, I or Br, and may be identical or different,
R₅ represents H, NO₂, CF₃, CN, OCH₃, F, I, Cl, Br, SO₃H or CO₂H in the meta or para position.
R represents a group such that the O-R bond can be hydrolysed by an enzyme.

7. Enzymatic substrate according to Claim 6, **characterized in that**
R₁ and R₂ together represent a fused benzene ring,
R₃ and R₄ represent a hydrogen atom,
R₅ represents H,
R represents a residue of the α- or β-sugar type, preferentially β-D-glucose or β-D-galactose.

8. Enzymatic substrate according to any one of Claims 1 to 3, of general formula (IVa): 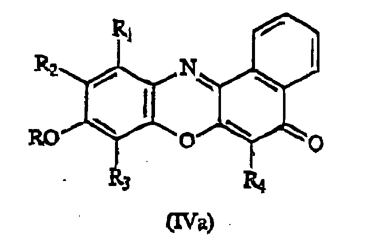 in which,
R₁ and R₂ each represent, independently of one another, H, Cl, F, I or Br, and may be identical or different, or R₁ and R₂ together represent a fused benzene ring which may or may not be substituted. Preferentially, R₁ represents H and R₂ represents Cl,
R₃ and R₄ each represent, independently of one another, H, Cl, F, I or Br, and may be identical or different. Preferentially R₃ and R₄ represent H,
R represents a group such that the O-R bond can be hydrolysed by an enzyme. In particular, R represents a residue of the α- or β-sugar type, preferentially β-D-glucoee or β-D-galactose.

9. Enzymatic substrate according to any one of Claims 1 to 3, of general formula (IVb): 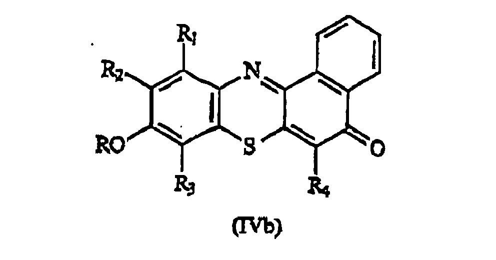 in which,
R₁ and R₂ each represent, independently of one another, H, Cl, F, I or Br, and may be identical or different, or R₁ and R₂ together represent a fused benzene ring which may or may not be substituted. Preferentially, R₁ represents H and R₂ represents Cl,
R₃ and R₄ each represent, independently of one another, H, Cl, F, I or Br, and may be identical or different. Preferentially, R₃ and R₄ represent H,
R represents a group such that the O-R bond can be hydrolysed by an enzyme. In particular, R represents a residue of the α- or β-sugar type, preferentially β-D-glucose or β-D-galactose.

10. Enzymatic substrate according to any one of Claims 1 to 3, of general formula (IVc): 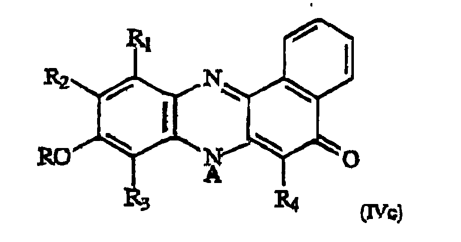 in which,
A represents an aryl or alkyl group,
R₁ and R₂ each represent, independently of one another, H, Cl, F, I or Br, and may be identical or different, or R₁ and R₂ together represent a fused benzene ring which may or may not be substituted. Preferentially, R₁ represents H and R₂ represents Cl,
R₃ and R₄ each represent, independently of one another, H, Cl, F, I or Br, and may be identical or different. Preferentially, R₃ and R₄ represent H,
R represents a group such that the O-R bond can be hydrolysed by an enzyme. In particular, R represents a residue of the α- or β-sugar type, preferentially β-D-glucose or β-D-galactose.

11. Enzymatic substrate according to Claim 8,
**characterized in that** ,
R₁ represents H and R₂ represents Cl,
R₃ and R₄ represent H,
R represents a residue of the α- or β-sugar type, preferentially β-D-glucose or β-D-galactose.

12. Enzymatic substrate according to Claim 10, **characterized in that**,
R₁, R₃ and R₄ represent H,
A represents a phenyl,
R represents a residue of the α- or β-sugar type, preferentially β-D-glucose or β-D-galactose.

13. Method for synthesizing an enzymatic substrate according to Claim 1, **characterized in, that** an intermediate compound is prepared, which is of general formula (V) 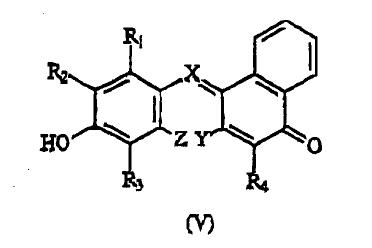 in which
X represents a nitrogen atom or a carbon atom substituted with a phenyl group, said phenyl group optionally being substituted in the meta or para position,
Y and Z represent a hydrogen atom when X represents a carbon atom, or Y and Z together represent an ether, thioether or amine bond optionally substituted with an alkyl or aryl group,
R₁ and R₂ each represent, independently of one another, H, Cl, F, I or Br, and may be identical or different, or R₁ and R₂ together represent a fused benzene ring which may or may not be substituted,
R₃ and R₄ each represent, independently of one another, H, Cl, F, I or Br, and may be identical or different, and a suitably protected group R is grafted onto the hydroxyl.

14. Method according to Claim 13, **characterized in that** the intermediate compound corresponds to the following formula (Va)

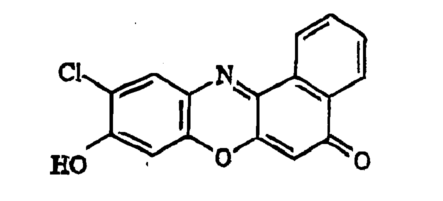

15. Method according to Claim 13, **characterized in that** the intermediate compound corresponds to the following formula (Vb)

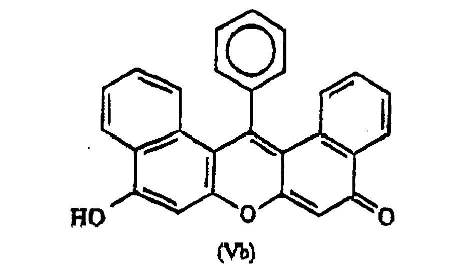

16. Method according to claim 13, **characterized in that** the intermediate compound corresponds to the following formula (vc)

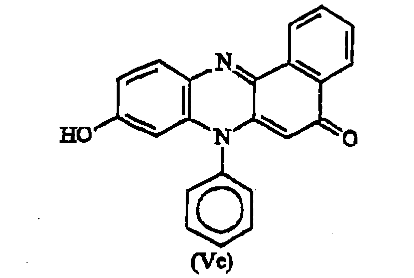

17. Composition for detecting and/or identifying and/or quantifying at least one microorganism, comprising at least one enzymatic substrate according to any one of Claims 1 to 12 and a reaction medium suitable for said microorganism(s).

18. Composition according to Claim 17, **characterized in that** said composition comprises at least two substrates according to any one of Claims 1 to 12, and the enzymatic hydrolysis of which produces different coloured and/or fluorescent products.

19. The composition according to Claim 17 or 18, **characterized in that** it also comprises another enzymatic substrate, which is different from the substrates according to any one of Claims 1 to 12, and the enzymatic hydrolysis of which produces a coloured or fluorescent product which is different from that released by the enzymatic hydrolysis of the substrate used according to Claim 17, or from those released by the enzymatic hydrolysis of the substrates used according to Claim 18.

20. Composition according to Claim 17, 18 or 19, **characterized in that** the reaction medium is solid, semi-solid or liquid.

21. Composition according to any one of Claims 17 to 20, **characterized in that** the concentration of the enzymatic substrate in the reaction medium is between 0.02 and 1 g/l, advantageously between 0.03 and 0.30 g/l, and preferentially between 0.04 and 0.10 g/l.

22. Diagnostic kit comprising a composition according to any one of Claims 17 to 21 and a container for the reaction medium.

23. Method for detecting and/or identifying and/or quantifying at least one microorganism in a specimen, **characterized in that**:
the microorganism originating from the specimen is brought into contact with a reaction medium containing an enzymatic substrate as defined in claims 1 to 12 so as to obtain an inoculating medium, and the coloured or fluorescent product formed by the hydrolysis of said enzymatic substrate is detected.

24. Method according to claim 23, **characterized in that** the inoculated reaction medium is incubated in a controlled atmosphere, for instance under aerobic, anaerobic or microaerophilic conditions or under an atmosphere of CO₂, preferentially under aerobic, microaerophilic and under CO₂ conditions.

25. Use of an enzymatic substrate according to any one of Claims 1 to 12, for detecting an analyte in techniques which use an enzymatic activity, such as the ELISA technique, in which the analyte is an antibody, an antigen or a nucleic acid, or molecular biology techniques in which the analyte is a gene of the β-galactosidase type.

## Patentansprüche

1. Enzymatisches Substrat mit der allgemeinen Formel (I): 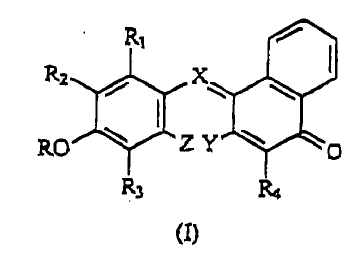 in welcher
X ein mit einer Phenylgruppe substituiertes Stickstoff- oder Kohlenstoffatom darstellt, wobei die Phenylgruppe wahlweise in meta- oder para-Position substituiert ist;
Y und Z ein Wasserstoffatom darstellen, wenn X ein Kohlenstoffatom darstellt, oder Y und Z gemeinsam eine Ether-, Thioether- oder eine Amin-Bindung darstellen, wahlweise substituiert mit einer Alkyl- oder Arylgruppe;
R₁ und R₂ jeweils unabhängig voneinander H, Cl, F, I, Br darstellen und identisch oder unterschiedlich sein können, oder R₁ und R₂ gemeinsam einen substituierten oder unsubstituierten fusionierten Benzolring darstellen;
R₃ und R₄ jeweils unabhängig voneinander H, Cl, F, I, Br darstellen und identisch oder unterschiedlich sein können;
R eine Gruppe darstellt, derart, dass die O-R-Bindung durch ein Enzym hydrolysierbar ist.

2. Enzymatisches Substrat gemäß Anspruch 1, **dadurch gekennzeichnet, dass** das Enzym ein Enzym der Familie der -Osidasen ist, wie β-Glucuronidase, β-Galactosidase, β-Glucosidase, 6-Phosphogalactohydrolase, α-Galactosidase, α-Amylase, α-Glucosidase, Hexosaminidasen wie N-Acetyl-β-glucosaminidase oder N-Acetyl-β-galactosaminidase, ein Enzym der Familie der Esterasen, der Lipasen, der Phosphatasen, der Sulfatasen, der DNasen, der Peptidasen und der Proteasen.

3. Enzymatisches Substrat nach einem der Ansprüche 1 oder 2, **dadurch gekennzeichnet, dass** R ein Rest vom Typ eines α-oder β-Zuckers ist, vorzugsweise β-D-Glucose oder β-D-Galactose oder ein β-D-Glucuronat oder ein Phosphat, ein Sulfat, ein Acetat.

4. Enzymatisches Substrat nach einem der Ansprüche 1 bis 3 der allgemeinen Formel (II): 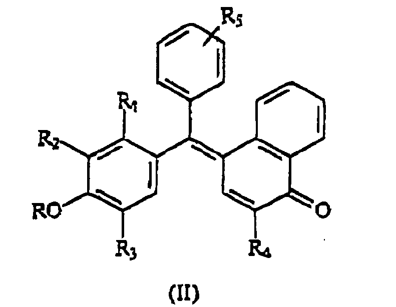 in welcher
R₁ und R₂ jeweils unabhängig voneinander H, Cl, F, I, Br darstellen und identisch oder unterschiedlich sein können, oder R₁ und R₂ jeweils einen substituierten oder unsubstituierten fusionierten Benzolring darstellen,
R₃ und R₄ jeweils unabhängig voneinander H, Cl; F, I, Br darstellen und identisch oder unterschiedlich sein können,
R₅ H, NO₂, CF₃, CN, OCH₃, F, I, Cl, Br, SO₃H, CO₂H in meta- oder para-Position darstellt, und
R eine Gruppe darstellt, derart, dass die O-R-Bindung durch ein Enzym hydrolysierbar ist.

5. Enzymatisches Substrat nach Anspruch 4, **dadurch gekennzeichnet, dass**
R₁ und R₂ gemeinsam einen fusionierten Benzolring darstellen,
R₃ und R₄ ein Wasserstoffatom darstellen,
R₅ ein Wasserstoffatom darstellt,
R einen Rest vom Typ eines α- oder β-Zuckers darstellt, vorzugsweise β-D-Glucose oder β-D-Galactose, oder ein Sulfat oder ein Acetat.

6. Enzymatisches Substrat nach einem der Ansprüche 1 bis 3 der allgemeinen Formel (III): 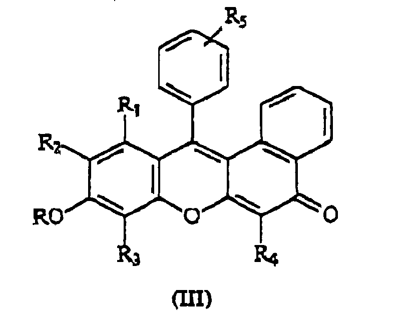 in welcher
R₁ und R₂ jeweils unabhängig voneinander H, Cl, F, I, Br darstellen und welche identisch oder unterschiedlich sein können, oder R₁ und R₂ gemeinsam einen substituierten oder unsubstituierten fusionierten Benzolring darstellen,
R₃ und R₄ jeweils unabhängig voneinander H, Cl, F, I, Br darstellen und identisch oder unterschiedlich sein können,
R₅ H, NO₂, CF₃, CN, OCH₃, F, I, Cl, Br, SO₃H, CO₂H an meta- oder para-Position, und
R eine Gruppe darstellt, derart, dass die O-R-Bindung durch ein Enzym hydrolysierbar ist.

7. Enzymatisches Substrat nach Anspruch 6, **dadurch gekennzeichnet, dass** R₁ und R₂ gemeinsam einen fusionierten Benzolring darstellen,
R₃ und R₄ ein Wasserstoffatom darstellen,
R₅ H darstellt,
R einen Rest vom Typ eines α- oder β-Zuckers darstellt, vorzugsweise β-D-Glucose oder β-D-Galactose.

8. Enzymatisches Substrat nach einem der Ansprüche 1 bis 3 mit der allgemeinen Formel (IVa): 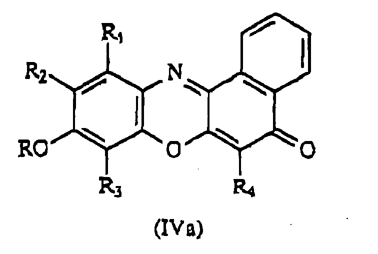 in welcher
R₁ und R₂ jeweils unabhängig voneinander H, Cl, F, I, Br darstellen und identisch oder unterschiedlich sein können, oder R₁ und R₂ einen substituierten oder unsubstituierten fusionierten Benzolring darstellen, vorzugsweise stellt R₁ H dar und R₂ Cl;
R₃ und R₄ jeweils unabhängig voneinander H, Cl, F, I, Br darstellen und identisch oder unterschiedlich sein können, vorzugsweise stellen R₃ und R₄ H dar;
R eine Gruppe darstellt, derart, dass die O-R-Bindung durch ein Enzym hydrolysierbar ist, insbesondere stellt R einen Rest vom Typ eines α- oder β-zuckers dar, vorzugsweise β-D-Glucose oder β-D-Galactose**.**

9. Enzymatisches Substrat nach einem der Ansprüche 1 bis 3 der allgemeinen Formel (IVb) 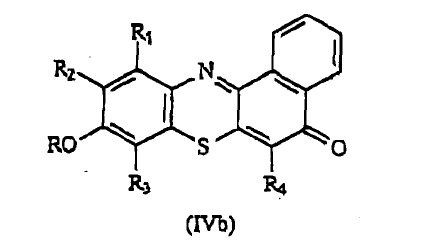 in welcher
R₁ und R₂ jeweils unabhängig voneinander H, Cl, F, I, Br darstellen und identisch oder unterschiedlich sein können, oder R₁ und R₂ einen substituierten oder unsubstituierten fusionierten Benzolring darstellen, vorzugsweise stellt R₁ H dar und R₂ Cl,
R₃ und R₄ jeweils unabhängig voneinander H, Cl, F, I, Br darstellen und identisch oder unterschiedlich sein können, vorzugsweise stellen R₃ und R₄ H dar;
R eine Gruppe darstellt, derart, dass die O-R-Bindung durch ein Enzym hydrolysierbar ist, insbesondere stellt R einen Rest vom Typ eines α- oder β-Zuckers dar, vorzugsweise β-D-Glucose oder β-D-Galactose.

10. Enzymatisches Substrat nach einem der Ansprüche 1 bis 3 der allgemeinen Formel (IVc) : 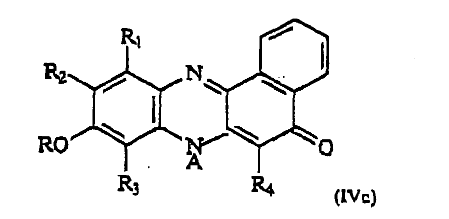 in welcher
A eine Aryl- oder Alkylgruppe darstellt,
R₁ und R₂ jeweils unabhängig voneinander H, Cl, F, I, Br darstellen und identisch oder unterschiedlich sein können, oder R₁ und R₂ einen substituierten oder unsubstituierten fusionierten Benzolring darstellen, vorzugsweise stellt R₁ H dar und R₂ Cl,
R₃ und R₄ jeweils unabhängig voneinander H, Cl, F, I, Br darstellen und identisch oder unterschiedlich sein können, vorzugsweise stellen R₃ und R₄ H dar;
R eine Gruppe darstellt, derart, dass die O-R-Bindung durch ein Enzym hydrolysierbar ist, insbesondere stellt R einen Rest vom Typ eines α- oder β-zuckers dar, vorzugsweise β-D-Glucose oder β-D-Galactose.

11. Enzymatisches Substrat nach Anspruch 8, **dadurch gekennzeichnet, dass**
R₁ H darstellt und R₂ Cl,
R₃ und R₄ H darstellen,
R einen Rest vom Typ eines α- oder β-Zuokers darstellt, vorzugsweise β-D-Glucoae oder β-D- Galactose.

12. Enzymatisches Substrat nach Anspruch 10, **dadurch gekennzeichnet, dass**
R₁, R₃ und R₄ H darstellen,
A ein Phenyl darstellt,
R einen Rest vom Typ eines α- oder β-Zuckers darstellt, vorzugsweise β-D-Glucose oder β-D- Galactose.

13. Verfahren zur Synthese eines enzymatischen Substrats gemäß Anspruch 1, **gekennzeichnet durch** die folgenden Schritte:
- Herstellen einer Zwischenverbindung mit der allgemeinen Formel (V) 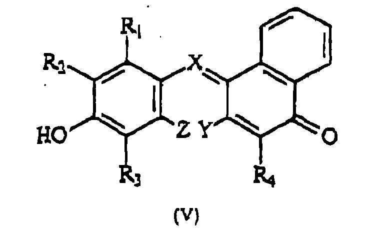
in welcher
X ein mit einer Phenylgruppe substituiertes Stickstoff- oder Kohlenstoffatom darstellt, wobei die Phenylgruppe wahlweise an meta- oder para-Position substituiert ist,
Y und Z ein Wasserstoffatom darstellen, wenn X ein Kohlenstoffatom darstellt oder Y und Z gemeinsam eine Ether-, Thioether, oder Amin-Bindung darstellen, wahlweise substituiert durch eine Alkyl- oder Arylgruppe,
R₁ und R₂ jeweils unabhängig voneinander H, Cl, F, I, Br darstellen und identisch oder unterschiedlich sein können, oder R₁ und R₂ einen substituierten oder unsubstituierten fusionierten Benzolring darstellen,
R₃ und R₄ jeweils unabhängig voneinander H, Cl, F, I, Br darstellen und identisch oder unterschiedlich sein können, und
Anfügen einer Gruppe R, passenderweise einer Schutzgruppe, an die Hydroxylgruppe,

14. Verfahren nach Anspruch 13, **dadurch gekennzeichnet, dass** die Zwischenverbindung der folgenden Formel (Va) entspricht

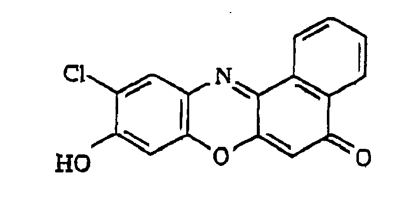

15. Verfahren nach Anspruch 13, **dadurch gekennzeichnet, dass** die Zwischenverbindung der folgenden Formel (Vb) entspricht

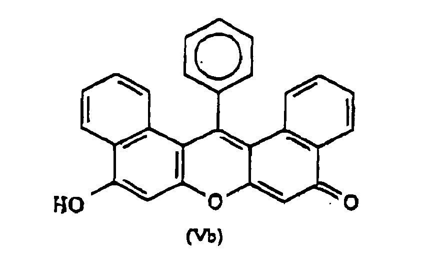

16. Verfahren nach Anspruch 13, **dadurch gekennzeichnet, dass** die Zwischenverbindung der folgenden Formel (Vc) entspricht

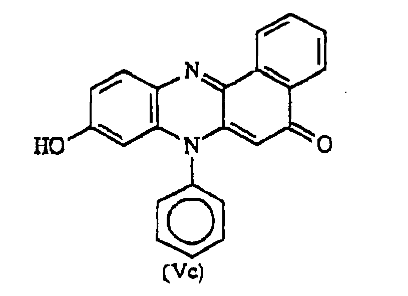

17. Zusammensetzung für die Detektion und/oder Identifizierung und/oder Quantifizierung von zumindest einem Mikroorganismus, wobei die Zusammensetzung zumindest ein enzymatisches Substrat nach einem der Ansprüche 1 bis 12 aufweist und ein für den oder die Mikroorganismen geeignetes Reaktionsmedium.

18. Zusammensetzung nach Anspruch 17, **dadurch gekennzeichnet, dass** die Zusammensetzung zumindest zwei Substrate gemäß einem der Ansprüche 1 bis 12 umfasst und deren enzymatische Hydrolyse zu Produkten führt, die sich hinsichtlich Farbe und/oder Fluoreszenz unterscheiden.

19. Zusammensetzung gemäß Anspruch 17 oder 18, **dadurch gekennzeichnet, dass** sie zusätzlich ein weiteres enzymatisches Substrat aufweist, welches sich von den Substraten gemäß einem der Ansprüche 1 bis 12 unterscheidet und dessen enzymatische Hydrolyse zu einem Produkt führt, das sich hinsichtlich Farbe oder Fluoreszenz von demjenigen unterscheidet, welches durch enzymatische Hydrolyse des Substrats freigesetzt wird, welches gemäß Anspruch 17 verwendet wird, oder von denjenigen, die durch enzymatische Hydrolyse der Substrate freigesetzt werden, welche gemäß Anspruch 18 verwendet werden.

20. Zusammensetzung gemäß Anspruch 17, 18 oder 19, **dadurch gekennzeichnet, dass** das Reaktionsmedium fest, halbfest oder flüssig ist.

21. Zusammensetzung nach einem der Ansprüche 17 bis 20, **dadurch gekennzeichnet, dass** die Konzentration des enzymatischen Substrate in dem Reaktionsmedium zwischen 0,02 und 1 g/l beträgt, vorteilhafterweise zwischen 0,03 und 0,30 g/l, und vorzugsweise zwischen 0,04 und 0,10 g/l.

22. Diagnose-Kit mit einer Zusammensetzung nach einem der Ansprüche 17 bis 21 und mit einem Behälter für das Reaktionsmedium.

23. Verfahren zur Detektion und/oder Identifizierung und/oder Quantifizierung von zumindest einem Mikroorganismus in einer Probe, **gekennzeichnet durch** die folgenden Schritte:
- In-Kontakt-Bringen des in einer Probe vorliegenden Mikroorganismus mit einem Reaktionsmedium, welches ein enzymatisches Substrat enthält, wie es in den Ansprüchen 1 bis 12 beschrieben ist, um ein beimpftes Medium zu erhalten, und
- Detektieren des farbigen oder fluoreszierenden Produktes, welches **durch** Hydrolyse des enzymatischen substrats gebildet wird.

24. Verfahren nach Anspruch 23, **dadurch gekennzeichnet, dass** das beimpfte Reaktionsmedium unter einer kontrollierten Atmosphäre inkubiert wird, wie bspw. unter aeroben, anaeroben, mikroaerophilen Bedingungen oder unter CO₂-Atmosphäre, vorzugsweise unter aeroben, mikroaerophilen und CO₂-Bedingungen.

25. Verwendung eines enzymatischen Substrats nach einem der Ansprüche 1 bis 12 zur Detektion eines Analyten in Verfahren, bei welchen eine enzymatischen Aktivität zum Einsatz kommt, wie das ELISA-Verfahren, in welchem der Analyt ein Antikörper oder ein Antigen oder eine Nucleinsäure ist, oder molekularbiologische Verfahren, in welchen der Analyt ein Gen vom Typ der β-Galactosidase ist.
